# EUROPEAN PATENT SPECIFICATION

(11) **EP 2 214 628 B1**
(45) Date of publication and mention of the grant of the patent: **19.10.2011**
(21) Application number: 08846097.7
(22) Date of filing: 21.10.2008
(51) Int. Cl.: A61K 8/24, A61K 8/34, A61K 8/37, A61K 8/60, A61K 8/73, A61K 8/81, A61K 8/92, A61K 31/202, A61Q 11/00, A61K 8/36, A23L 1/30, A23L 1/22

(54) **ORODISPERSIBLE COMPOSITION COMPRISING POLYUNSATURATED FATTY ACIDS WITHOUT BAD ODOUR OR TASTE**
IM MUND DISPERGIERBARE ZUSAMMENSETZUNG MIT MEHRFACH UNGESÄTTIGTEN FETTSÄUREN OHNE SCHLECHTEN GERUCH ODER GESCHMACK
COMPOSITION ORODISPERSIBLE COMPRENANT DES ACIDES GRAS POLYINSATURÉS ET NE PRÉSENTANT NI ODEUR NI GOÛT DÉSAGRÉABLE

(30) Priority: 30.10.2007 EP 07291305
(43) Date of publication of application: 11.08.2010
(73) Proprietor: Bayer Consumer Care AG, 4052 Basel (CH)
(72) Inventor: KABARADJIAN, Catherine, F-74100 Vétraz-Monthoux (FR)
(74) Representative: Albers, Markus
(86) International application number: PCT/EP2008/008883
(87) International publication number: WO 2009/056248

(56) References cited:
- EP-A- 0 276 772
- US-A1- 2005 106 240
- DATABASE WPI Week 199650 Thomson Scientific, London, GB; AN 1996-502976 XP002513252 & JP 08 259943 A (NIPPON SUISAN KAISHA LTD) 8 October 1996 (1996-10-08)
- BENATTI P. ET AL: "Polyunsaturated Fatty Acids: Biochemical, Nutritional and Epigenetic Properties" JOURNAL OF THE AMERICAN COLLEGE OF NUTRITION, [Online] vol. 23, no. 4, 2004, pages 281-302, XP002513251 Retrieved from the Internet: URL:http://www.jacn.org/cgi/reprint/23/4/2 81> [retrieved on 2009-02-02]
- SERFERT, Y ET AL: "Chemical stabilisation of oils rich in long-chain polyunsaturated fatty acids during homogenisation, microencapsulation and storage" FOOD CHEMISTRY, [Online] vol. 113, no. 2009, pages 1106-1112, XP002513348 Retrieved from the Internet: URL:http://www.sciencedirect.com/science?_ ob=MImg&_imagekey=B6T6R-4TBXGHF-6-3&_cdi=5 037&_user=987766&_orig=search&_coverDate=0 4%2F15%2F2009&_sk=998869995&view=c&wchp=dG LbVzb-zSkWA&md5=498b74eaab9493c42456fd2267 bdc4f4&ie=/sdarticle.pdf> [retrieved on 2009-02-02]

## Description

The present invention relates to an orodispersible composition without bad odour, smell or taste comprising polyunsaturated fatty acids (PUFA), its process for preparation and its use as nutritional supplement or as dietary supplement for balancing the blood lipid level, preventing or reducing the risk of the development of atherosclerotic changes, disorders or diseases.

Polyunsaturated fatty acids PUFA) are the active ingredients e.g. in fish oil and are responsible for significant low blood lipid levels and low incidence of hypertension which was shown in a epidemiological study with Inuits (M.H. Davidson, P. R. Liebson, Cardiovascular Reviews&Reports 1986, 7, 461-472). In particular the blood concentration of the low density lipoprotein cholesterol (LDL) is lowered and that of the high density lipoprotein cholesterol (HDL) is increased. Coronary heart disease (CHD) is the major cause of death in the western countries and high plasma cholesterol levels, more specifically the LDL/HDL ratio, is highly correlated with the risk of CHD (Willett and Sacks, N. Eng. J. Med. 1991, 121, 324).

The PUFA found in fish oil have carbon chains of 18, 20 or 22 atoms and can be classified in n-3 omega and n-6 omega fatty acids which are essential for the human body. In particular the omega-3 fatty acids eicosapentenoic acid (EPA) and docosahexenoic acid (DHA) are only found in fish and other marine life. Fish oil is therefore the most important food source for omega-3 fatty acids.

Standard nutritional supplement of PUFA is achieved by a daily administration of 500-1000 mg liquid fish oil. The fish oil is normally administered in capsules in order to prevent the fishy smell and odour. Nevertheless some people experience gastrointestinal upset of the fishy smell even hours after the fish oil is taken. The reason is that after decomposition of the galantine wall in the gastro-intestinal tract, the voluminous dosage of the fish oil is released as a macroscopic drop which interfere with the resorption of the fish oil. The problems of bad smell, odour and resorption can be solved in part by microdispersed fish oil preparations as pulverulent or aqueous matrix as described in EP 0 276 772. However, even the microdispersed fish oil granule or powder has a remaining fishy smell when pressed in simple tablets, so that flavours or antioxidants are needed to be added to the formulations.

The flavours are used to domineer over the fishy smell of the fish oil as described e.g. in JP 08092587 or JP 08228678. Taste masking is also achieved by adding milk products to the fish oil formulation as described e.g. in US 2003/0198728 or JP 2002-204656 or by coating or encapsulating the PUFA as described e.g. in WO 2004/016720 and WO 2005/029978.

The reason for the fishy smell of the fish oil is based on the oxidation of the unsaturated part of the PUFA. In order to prevent oxidation and to stabilize the PUFA antioxidants e.g. tocopherol can be added to the formulation as described e.g. in DE 20105126 or EP 1 155 620.

JP 08259943 relates to the preparation of nutritional supplements or food comprising fish oil, said compositions lacking the fishy or bad smell, odor or taste. It discloses the use of sugar alcohols as active ingredient for preventing the apparition of bad taste in emulsified oils and fats and their dried powder compositions. To this end, first the emulsions are prepared to which the sugar alcohol is added and dissolved in order to thoroughly mix the antioxidative compound with the labile oil or fat. The emulsion is proved to be stable as well as a freeze-dried powder obtained from the same (working examples, paragraphs 15 to 21).

The objective of the present invention is to provide an alternative fast dissolving and entirely taste masked fish or perilla oil or PUFA comprising composition with easier accessibility, manufacture and handling while still achieving complete taste masking and short disintegration times.

Surprisingly it is found that a bad or fishy smell of the composition according to the present invention can be avoided.

Subject of the present invention is an orodispersible composition comprising
i) fish oil, perilla oil, or polyunsaturated fatty acids (PUFA) as omega-3 fatty acids, omega-6-fatty acids, arachidonic acid, linoleic acid, alpha-linoleic acid, dihomogammalinoleic acid, eicosapentenoic acid (EPA), docosahexenoic acid (DHA) or mixtures thereof and
ii) a disintegrant which is a solid dispersion comprising mannitol, xylitol, microcrystalline cellulose, crospovidone and dibasic calcium phosphate,
   whereby the oral composition has not a fishy or bad smell, odour or taste.

According to the present invention polyunsaturated fatty acids (PUFA) include, but are not limited to, fish oil, perilla oil, omega-3 fatty acids, omega-6-fatty acids, arachidonic acid, linoleic acid, alpha-linoleic acid, dihomogammalinoleic acid, eicosapentenoic acid (EPA), docosahexenoic acid (DHA) and mixtures thereof. Preference is given to fish oil, omega-3 fatty acids, eicosapentenoic acid (EPA), docosahexenoic acid (DHA) and mixtures thereof. Most preferably fish oil containing eicosapentenoic acid (EPA) and docosahexenoic acid (DHA) is used. In a preferred embodiment the composition according to the invention comprises PUFA, in particular fish oil powder or granulate, in an amount of 5 % to 70%, more preferable 40 % to 60 % by weight of the composition. The total amount of the PUMA, in particular fish oil powder or granulate, used in the composition is 100 mg - 1000 mg, preferably 400 mg - 900 mg.

In a most preferred embodiment the composition according to the invention comprises eicosapentenoic acid (EPA) in an amount of 0.5 % to 10 %, preferably 1.5 % to 4 % by weight of the composition or in an amount of 1 % to 20 %, preferably 3 % to 8 % by weight of the fish oil powder or granulate contained in the composition. The total amount of EPA in the composition is 10 mg-100 mg, preferably 15 mg- 50 mg.

In a most preferred embodiment the composition according to the invention comprises docosahexenoic acid (DHA) in an amount of 0.5 % to 10 %, preferably 1 % to 4 % by weight of the composition or in an amount of 1 % to 20 %, preferably 2 % to 6 % by weight of the fish oil powder or granulate contained in the composition. The total amount of DHA in the composition is 10 mg - 50 mg, preferably 15 mg - 30 mg.

The PUFA, in particular the fish oil containing eicosapentenoic acid (EPA) and docosahexenoic acid (DHA), is used preferably in a microdispersed form as a granulate or powder, in a pulverulent or aqueous matrix as described in EP 0 276 772. Preference is given to a fish oil granulate or powder.

In a preferred embodiment the pulverulant matrix of the fish oil powder or granulate comprises at least a homogenous protective colloid, a surfactant, optionally a diluent, stabilizer and further pharmaceutical excipients. In the case of the aqueous matrix the mentioned ingredients are used as an aqueous solution.

The protective colloids of the pulverulant matrix include but are not limited to polypeptides such as gelatine, casein, caseinate, polysaccharides such as starch, dextrin, pectin, Arabic gum, milk, milk powder, polyvinyl alcohols, polyvinylpyrrolidone, vinylpyrrolidone-vinylacetate-copolymers, acrylic acid- and methacrylic acid-copolymers with acrylic acid- or methacrylic acid esters, methyl cellulose, carboxymethyl cellulose, hydroxypropyl cellulose, alginates or mixtures thereof.

Diluents of the pulverulant matrix include but are not limited to sugar or sugar alcohols e.g. saccharose, lactose, invert sugar, sorbit, mannit or glycerine.

Stabilizers of the pulverulant matrix include but are not limited to tocopherol, t-butyl hydroxytoluol, t-butyl hydroxyanisol and ethoxyquine.

Surfactants of the pulverulant matrix include but are not limited to esters of long chain fatty acids and ascorbic acid, esters of mono- and diglycerin and fatty acids and oxethylated derivatives thereof, esters of mono fatty acid glycerides with acetic acid, citric acid, lactic acid, diacetyltartrate, salts of 2-(2'-stearoyllactyl) lactic acid, polyglycerine fatty acid esters, sorbitan fatty acid esters, porpylenglycol-fatty acid esters, ascorbylpalmitate and lecithin.

The pulverulant matrix comprises fish oil in the amount of 5 % to 70 %, preferably 50 % to 60 %, one or more surfactants in an amount of 1 % to 30 %, preferably 5 % to 15 %, a protective colloid in an amount of 5 % to 50 %, preferably 10 % to 40 %, a diluent in an amount of 0 % to 70 %, preferably 3 % to 35 %, by weight of the dry mass of the fish oil powder or granulate.

In a preferred embodiment the pulverulant matrix comprises the fish oil in small particles having an average particle size of less then 10 µm, more preferably less then 1 µm, most preferably less then 0.5 µm.

The fish oil powder or granulate can be prepared as described in EP 0 276 772.

According to the present invention the solid dispersion responsible for rapid disintegration comprises mannitol, xylitol, microcrystalline cellulose, crospovidone and dibasic calcium phosphate wherein microcrystalline cellulose, crospovidone and dibasic calcium phosphate are dispersed in the mixture of mannitol and xylitol. The solid dispersion according to the invention can be prepared as described in EP 1523974.

The amount of mannitol and xylitol in the solid dispersion is from 40 % to 90 %, preferably from 50 % to 80 %, more preferably from 60 % to 78 %, and most preferably from 62 % to 78 % by weight of the solid dispersion, and the amount of mannitol and xylitol in the total composition is from 18 % to 41 %, preferably from 23 % to 36 %, more preferably from 27 % to 35 % , and most preferably from 28 % to 35 % by weight of the total composition.

The ratio by weight of mannitol and xylitol is from (98 to 67) : (2 to 33), preferably from (98 to 87) : (2 to 13), more preferably from (97 to 87) : (3 to 13), and most preferably from (96 to 89) : (4 to 11).

The amount of dibasic calcium phosphate (which corresponds to calcium monohydrogen phosphate) in the solid dispersion is from 1 % to 30 %, preferably 2 % to 15 %, and more preferably 3 % to 8 % by weight of the solid dispersion, and the amount of dibasic calcium phosphate in the total composition is from 0.4 % to 13 %, preferably 1 % to 7 %, and more preferably 2 % to 4 % by weight of the total composition.

The amount of microcrystalline cellulose in the solid dispersion is from 8 % to 22 %, preferably from 10 % to 22 %, more preferably from 12 % to 21 % by weight of the solid dispersion, and the amount of microcrystalline cellulose in the total composition is from 3 % to 10 %, preferably from 4 % to 10 %, more preferably from 5 % to 9 % by weight of the total composition.

The amount of crospovidone in the solid dispersion is from 5 % to 15 %, preferably from 5 % to 14 %, more preferably from 6 % to 13 % by weight of the solid dispersion, and the amount of crospovidone in the total composition is from 2 % to 7 %, preferably from 2 % to 6 %, more preferably from 3 % to 6 % by weight of the total composition.

In a preferred embodiment D-mannitol is used in the solid dispersion.

The composition according to the invention can comprise further active ingredients such as vitamins and minerals. Vitamins include, but are not limited to, vitamin A, beta carotene, vitamin C (ascorbic acid), vitamin D3 (cholecalcipherol), vitamin E (tocopherol acetate), vitamin B1 (thiamine), vitamin B2 (riboflavin), nicotinamide, vitamin B5 (panthothenic acid), vitamin B6 (pyridoxine), folic acid, vitamin B12 (cyanocobalamin), vitamin K1, vitamin K2, especially menaquinone 7-10, and biotin. Minerals include, but are not limited to, iron salts, copper salts, calcium salts such as calcium carbonate, calcium phosphate, calcium glycerophosphate; magnesium salts such as magnesium phosphate, magnesium sulphate (dihydrate) or magnesium oxide; zinc salts such as zinc citrate; selenium salts such as sodium selenate; potassium iodide; manganese salts such as manganese sulphate; molybdate salts such as sodium molybdate; chromium salts such as chromium chloride; sodium chloride and potassium chloride.

The composition according to the present invention can be used as nutritional supplement or as dietary supplement for balancing the blood lipid level, preventing or reducing the risk of the development of atherosclerotic changes, disorders or diseases in a patient. The inventive composition can also be used as nutritional or as dietary supplement for developing and maintaining the cognitive functions connected with e.g. eyes, memory, language etc. or for alleviating and/or preventing blood vessel diseases, cardiovascular, cerebrovascular and nervous diseases such as e.g. hypertension, cardiac infarction, Alzheimer, Parkinson and depression, or for alleviating hormonal, immunologic disorders or obesity. Furthermore, it can be used as nutritional or dietary supplement to support treatments of diabetes, cancer and/or inflammatory affections. A patient, for the purpose of this invention, is a mammal, including a human. The use as a nutritional or dietary supplement is especially preferred for pregnant women, children and elderly persons.

A further aspect of the invention is a method for balancing the blood lipid level, preventing or reducing the risk of the development of atherosclerotic changes, disorders or diseases, for developing and maintaining the cognitive functions connected with e.g. eyes, memory, language etc., for alleviating and/or preventing blood vessel diseases, cardiovascular, cerebrovascular and nervous diseases such as e.g. hypertension, cardiac infarction, Alzheimer, Parkinson and depression, or for alleviating hormonal, immunologic disorders or obesity or for supporting treatments of diabetes, cancer and/or inflammatory affections by administering the inventive composition as nutritional supplement or as dietary supplement to a patient which is, for the purpose of this invention, a mammal, including a human, especially pregnant women, children and elderly persons.

The composition according to the invention is administered orally one or more, preferably up to three, more preferably up to two times per day. With each administration the number of dosage forms taken in at the same time should not exceed two.

Nevertheless, it may in some cases be advantageous to deviate from the amounts specified, depending on body weight, individual behaviour toward the active ingredient, type of preparation and time or interval over which the administration is effected. For instance, less than the aforementioned minimum amounts may be sufficient in some cases, while the upper limit specified has to be exceeded in other cases.

Ingredients of the oral dosage form are those which are accepted for pharmaceuticals and nutritional supplements and physiologically unobjectionable, for example: as fillers cellulose derivatives (e.g. microcrystalline cellulose), sugars (e.g. lactose), sugar alcohols (e.g. mannitol, sorbitol), inorganic fillers (e.g. calcium phosphates), binders (e.g. polyvinylpyrrolidone, gelatin, starch derivatives and cellulose derivatives), and all other excipients required to produce formulations of pharmaceuticals and nutritional supplements of the desired properties, e.g. lubricants (magnesium stearate), e.g. disintegrants (e.g. crosslinked polyvinylpyrrolidone, sodium carboxymethylcellulose), e.g. wetting agents (e.g. sodium lauryl sulphate), e.g. release-slowing agents (e.g. cellulose derivatives, polyacrylic acid derivatives), e.g. coloured pigments.

Excipients for pharmaceuticals and nutritional supplements familiar to the skilled person are also described for example in the following handbook: "Handbook of Pharmaceutical Excipients", Rowe R.C., Sheskey P.J. & Weller, P.J., American Pharmaceutical Association, Washington, 4th edition 2003.

The orodispersible tablet mentioned herein is produced by general standard processes. The solid dispersion is produced as described in EP 1 523 974. E.g. tablets can be produced by mixing and/or granulating the active ingredients together with the excipients (e.g. the whole solid dispersion part) to form a blend which is finally pressed to tablets. Optionally different blends containing different ingredients and excipients can be premixed and combined to a final blend which is then pressed to tablets.

Advantage of the composition of the present invention is that for the preparation of the composition a complex and expensive taste masking technology known in the prior art such as coating of tablets or granules, adding of antioxidants, or putting the PUFA into a capsule is not needed. The composition of the present invention can be prepared by simple and well-known standard procedures. Another well-known taste masking method is the addition of flavours in order to cover and mask the bad smell. This taste masking method is normally restricted to only a few applicable flavours which have to be selected in each case. However, flavouring ingredients are not needed for taste masking in the composition of the present invention.

Preference is given to an orodispersible composition which is not a coated tablet, coated granule, or capsule or which does not comprise an antioxidant or flavour or other taste masking substance.

Preference is given to a fast disintegrating orodispersible tablet, that means it disintegrates rapidly in the oral cavity. The disintegration time of the fast disintegrating orodispersible tablet is less equal than 100 sec., preferably less equal than 80 sec.

The composition according to the present invention shows a good and/or fast resorption of the PUFA after administration of the composition. Furthermore the composition facilitates a quick intake, optionally without water or drink.

The composition according to the invention shows an acceptable hardness and friability to be manufactured without affecting the beadlet integrity, i.e. no PUFA exudates from the tablet matrix.

### Examples:

### Example 1:

An orodispersible tablet consisting of:
- 500 mg of dry fish oil powder (omega-3, 18:12) including 23.5 mg of EPA and 16.5 mg of DHA
- 500 mg of F-melt type C
   and optionally the following excipients:

- 15 mg of anhydrous citric acid
- 10 mg of Aspartam

F-melt type C is a commercially available disintegrant and consists of crospovidone, mannitol, xylitol, dibasic calcium phosphate and microcrystalline cellulose (Fuji, Chemical Industry Co., Ltd., Japan) and can be prepared as described in EP 1 523 974.

### Example 2:

An orodispersible tablet consisting of:
- 890 mg of dry fish oil powder (omega-3, 18:12) including 41.83 mg of EPA and 29.37 mg of DHA
- 750 mg of F-melt type C
   and optionally the following flavours and excipients:
   - 25 mg of anhydrous citric acid
   - 35 mg of Aspartam
   - 5 mg of stearic acid
   - 5 mg of iron oxide (red)

F-melt type C is a commercially available disintegrant and consists of crospovidone, mannitol, xylitol, dibasic calcium phosphate and microcrystalline cellulose (Fuji, Chemical Industry Co., Ltd., Japan) and can be prepared as described in EP 1 523 974.

### Example 3:

An orodispersible tablet consisting of:
- 500 mg of dry fish oil powder (omega-3, 18:12) including 23.5 mg of EPA and 16.5 mg of DHA
- 500 mg of F-melt type C
   and optionally the following flavours and excipients:
   - 15 mg of anhydrous citric acid
   - 50 mg of Lemon flavour
   - 20 mg of honey flavour
   - 10 mg of Aspartam

F-melt type C is a commercially available disintegrant and consists of crospovidone, mannitol, xylitol, dibasic calcium phosphate and microcrystalline cellulose (Fuji, Chemical Industry Co., Ltd., Japan) and can be prepared as described in EP 1 523 974.

### Example 4:

An orodispersible tablet consisting of:
- 890 mg of dry fish oil powder (omega-3, 18:12) including 41.83 mg of EPA and 29.37 mg of DHA
- 750 mg of F-melt type C
   and optionally the following flavours and excipients:
   - 25 mg of anhydrous citric acid
   - 50 mg of Lemon flavour
   - 20 mg of honey flavour
   - 30 mg of pineapple flavour
   - 35 mg of Aspartam
   - 5 mg of stearic acid
   - 5 mg of iron oxide (red)

F-melt type C is a commercially available disintegrant and consists of crospovidone, mannitol, xylitol, dibasic calcium phosphate and microcrystalline cellulose (Fuji, Chemical Industry Co., Ltd., Japan) and can be prepared as described in EP 1 523 974.

### Manufacturing process for orodispersible tablets

### Direct compression process :

All ingredients are mixed together in a tumble mixer for 20 min. and optionally 5 min. before the end the lubricant, if any, (stearic acid) is added. The final blend is pressed into tablets with a rotary press.

### Results:

Examples 1 and 2 do not show any bad or fishy taste or smell, in examples 3 and 4 only small amounts of flavour are added to improve the taste of the otherwise savourless tablet.

The disintegration time is 40 ± 5 sec. for example 1 and 3 and 60 ± 5 sec. for example 2 and 4.

## Claims

1. Orodispersible composition comprising
i) fish oil, perilla oil, or polyunsaturated fatty acids (PUFA) as omega-3 fatty acids, omega-6-fatty acids, arachidonic acid, linoleic acid, alpha-linoleic acid, dihomogammalinoleic acid, eicosapentenoic acid (EPA), docosahexenoic acid (DHA) or mixtures thereof and
ii) a disintegrant which is a solid dispersion comprising mannitol, xylitol, microcrystalline cellulose, crospovidone and dibasic calcium phosphate, whereby the composition does not exhibit a fishy or bad smell, odour or taste

2. Composition of claim 1 , wherein the fish oil contains eicosapentenoic acid (EPA) and docosahexenoic acid (DHA).

3. Composition of claim 1 or 2, wherein the fish oil is fish oil powder or granulate.

4. Composition of any of claims 1 to 3, wherein the amount of the fish oil, perilla oil, or PUFA is of 5 % to 70% by weight of the composition.

5. Composition of any of claims 1 to 4, wherein the amount of the fish oil, perilla oil, or PUFA in the composition is 100 mg - 1000 mg.

6. Composition of any of claims 1 to 5 wherein in the solid dispersion microcrystalline cellulose, crospovidone and dibasic calcium phosphate are dispersed in the mixture of mannitol and xylitol.

7. Composition of any of claims 1 to 6 comprising at least one further mineral and/or vitamin.

8. Composition of any of claims 1 to 7 which is a fast disintegrating orodispersible tablet having a disintegration time of less equal than 100 sec..

9. Composition of any of claims 1 to 8 which is not a coated tablet, coated granule, or capsule.

10. Composition of any of claims 1 to 9 which does not comprise any antioxidant or flavour or other taste masking substance.

11. Process for manufacturing of the composition according to any of claims 1 to 10 by mixing and/or granulating the active ingredients together with the excipients to form a blend which is pressed into tablets.

12. Process for manufacturing of the composition according to any of claims 1 to 10 by
premixing different blends containing different ingredients and excipients,
combining to a final blend and
pressing into tablets.

13. Composition according to any of claims 1 to 10 as nutritional supplement or as dietary supplement.

14. Use of the composition according to claims 1 to 10 for the preparation of a nutritional supplement or dietary supplement for balancing the blood lipid level, preventing or reducing the risk of the development of atherosclerotic changes, disorders or diseases, for developing and maintaining the cognitive functions, for alleviating and/or preventing blood vessel diseases, cardiovascular, cerebrovascular and nervous diseases, for alleviating hormonal, immunologic disorders or obesity, for supporting treatments of diabetes, cancer and/or inflammatory affections.

15. Composition according to claims 1 to 10 for balancing the blood lipid level, preventing or reducing the risk of the development of atherosclerotic changes, disorders or diseases, for developing and maintaining the cognitive functions, for alleviating and/or preventing blood vessel diseases, cardiovascular, cerebrovascular and nervous diseases, for alleviating hormonal, immunologic disorders or obesity, for supporting treatments of diabetes, cancer and/or inflammatory affections

## Patentansprüche

1. Im Munde dispergierbare Zusammensetzung, umfassend
i) Fischöl, Perillaöl oder mehrfach ungesättigte Fettsäuren (PUFA) als omega-3-Fettsäuren, omega-6-Fettsäuren, Arachidonsäure, Linolsäure, alpha-Linolensäure, Dihomo-gamma-Linolensäure, Eicosapentensäure (EPA), Docosahexensäure (DHA) oder Mischungen davon, und
ii) ein Sprengmittel, bei dem es sich um eine feste Dispersion, umfassend Mannit, Xylit, mikrokristalline Zellulose, Crospovidon und dibasisches Calciumphosphat, handelt,
wobei die Zusammensetzung keinen fischartigen oder schlechten Geruch, Duft oder Geschmack aufweist.

2. Zusammensetzung nach Anspruch 1, wobei das Fischöl Eicosapentensäure (EPA) und Docosahexensäure (DHA) enthält.

3. Zusammensetzung nach Anspruch 1 oder 2, wobei es sich bei dem Fischöl um Fischölpulver oder-granulat handelt.

4. Zusammensetzung nach einem der Ansprüche 1 bis 3, wobei die Menge an Fischöl, Perillaöl oder PUFA 5 Gew.-% bis 70 Gew.-% der Zusammensetzung beträgt.

5. Zusammensetzung nach einem der Ansprüche 1 bis 4, wobei die Menge an Fischöl, Perillaöl oder PUFA in der Zusammensetzung 100 mg - 1000 mg beträgt.

6. Zusammensetzung nach einem der Ansprüche 1 bis 5, wobei in der festen Dispersion die mikrokristalline Zellulose, das Crospovidon und das dibasische Calciumphosphat in der Mischung aus Mannit und Xylit dispergiert sind.

7. Zusammensetzung nach einem der Ansprüche 1 bis 6, umfassend mindestens einen weiteren Mineralstoff und/oder ein weiteres Vitamin.

8. Zusammensetzung nach einem der Ansprüche 1 bis 7, bei der es sich um eine rasch verfallende im Mund dispergierbare Tablette mit einer Zerfallszeit von 100 Sekunden oder weniger handelt.

9. Zusammensetzung nach einem der Ansprüche 1 bis 8, bei der es sich nicht um eine Filmtablette, ein Filmgranulat oder eine Kapsel handelt.

10. Zusammensetzung nach einem der Ansprüche 1 bis 9, die kein Antioxidationsmittel oder kein Aroma oder keine andere geschmacksmaskierende Substanz umfasst.

11. Verfahren zur Herstellung der Zusammensetzung nach einem der Ansprüche 1 bis 10 durch Vermischen und/oder Granulieren der Wirkstoffe zusammen mit den Grundstoffen unter Bildung eines Blends, das zu Tabletten verpresst wird.

12. Verfahren zur Herstellung der Zusammensetzung nach einem der Ansprüche 1 bis 10 durch
Vormischen von verschiedenen Blends, die verschiedene Bestandteile und Grundstoffe enthalten,
Zusammenfügen, so dass man ein Abschluss-Blend erhält, und
Verpressen zu Tabletten.

13. Zusammensetzung nach einem der Ansprüche 1 bis 10 als Nahrungsmittelzusatzstoff oder als Diätzusatzstoff.

14. Verwendung der Zusammensetzung nach den Ansprüchen 1 bis 10 für die Herstellung eines Nahrungsmittelzusatzstoffs oder eines Diätzusatzstoffs zum Ausgleichen des Lipidspiegels im Blut, zum Vorbeugen oder Verringern des Risikos der Entwicklung von artheriosklerotischen Veränderungen, Störungen oder Krankheiten, zum Fördern und Aufrechterhalten der kognitiven Funktionen, zum Lindern und/oder zum Vorbeugen von Blutgefäßkrankheiten, Herz-Kreislauf Krankheiten, cerebrovaskulärer Verschlusskrankheit und Nervenkrankheiten, zum Lindern von Hormon-, immunologischen Störungen oder Fettleibigkeit, zum Unterstützen von Behandlungen von Diabetes, Krebs und/oder Entzündungserkrankungen.

15. Zusammensetzung nach den Ansprüchen 1 bis 10 zum Ausgleichen des Lipidspiegels im Blut, zum Vorbeugen oder Verringern des Risikos der Entwicklung von artheriosklerotischen Veränderungen, Störungen oder Krankheiten, zum Fördern und Aufrechterhalten der kognitiven Funktionen, zum Lindern und/oder zum Vorbeugen von Blutgefäßkrankheiten, Herz-Kreislauf-Krankheiten, cerebrovaskulärer Verschlusskrankheit und Nervenkrankheiten, zum Lindern von Hormon-, immunologischen Störungen oder Fettleibigkeit, zum Unterstützen von Behandlungen von Diabetes, Krebs und/oder Entzündungserkrankungen.

## Revendications

1. Composition orodispersible comprenant
i) de l'huile de poisson, de l'huile de périlla, ou des acides gras polyinsaturés (PUFA) tels que les acides gras oméga-3, les acides gras oméga-6, l'acide arachidonique, l'acide linoléique, l'acide alpha-linoléique, l'acide dihomogammalinoléique, l'acide eicosapenténoïque (EPA), l'acide docosahexénoïque (DHA) ou des mélanges de ceux-ci, et
ii) un délitant qui est une dispersion solide comprenant du mannitol, du xylitol, de la cellulose microcristalline, de la crospovidone et du phosphate de calcium dibasique,
la composition ne sentant pas le poisson ou mauvais, n'ayant pas d'odeur de poisson ou de mauvaise odeur, ou n'ayant pas de goût de poisson ou de mauvais goût.

2. Composition selon la revendication 1, **caractérisée en ce que** l'huile de poisson contient de l'acide eicosapenténoïque (EPA) et de l'acide docosahexénoïque (DHA).

3. Composition selon la revendication 1 ou 2, **caractérisée en ce que** l'huile de poisson est de la poudre ou du granulé d'huile de poisson.

4. Composition selon l'une quelconque des revendications 1 à 3, **caractérisée en ce que** la quantité d'huile de poisson, d'huile de périlla, ou de PUFA est de 5 % à 70 % en poids de la composition.

5. Composition selon l'une quelconque des revendications 1 à 4, **caractérisée en ce que** la quantité d'huile de poisson, d'huile de périlla, ou de PUFA dans la composition est de 100 mg - 1000 mg.

6. Composition selon l'une quelconque des revendications 1 à 5, **caractérisée en ce que** dans la dispersion solide, la cellulose microcristalline, la crospovidone et le phosphate de calcium dibasique sont dispersés dans le mélange de mannitol et de xylitol.

7. Composition selon l'une quelconque des revendications 1 à 6, comprenant au moins un autre minéral et/ou une autre vitamine.

8. Composition selon l'une quelconque des revendications 1 à 7, **caractérisée en ce qu'**il s'agit d'un comprimé orodispersible à désintégration rapide ayant un temps de désintégration inférieur ou égal à 100 sec.

9. Composition selon l'une quelconque des revendications 1 à 8, **caractérisée en ce qu'**il ne s'agit pas d'un comprimé pelliculé, d'un granulé pelliculé ou d'une capsule.

10. Composition selon l'une quelconque des revendications 1 à 9, **caractérisée en ce qu'**elle ne comprend pas d'antioxydant ou d'arôme ou autre correcteur du goût.

11. Procédé de fabrication de la composition selon l'une quelconque des revendications 1 à 10, par mélange et/ou granulation des principes actifs avec les excipients pour former un mélange qui est mis en comprimés.

12. Procédé de fabrication de la composition selon l'une quelconque des revendications 1 à 10, par
prémélange de différents mélanges contenant différents ingrédients et excipients,
combinaison en un mélange final, et
mise en comprimés.

13. Composition selon l'une quelconque des revendications 1 à 10, en tant que complément nutritionnel ou complément diététique.

14. Utilisation de la composition selon les revendications 1 à 10, pour la préparation d'un complément nutritionnel ou d'un complément diététique destiné à équilibrer le taux de lipides sanguin, prévenir ou réduire le risque de développement de changements, de troubles ou de maladies athérosclérotiques, développer et maintenir les fonctions cognitives, soulager et/ou prévenir les maladies des vaisseaux sanguins, les maladies cardiovasculaires, cérébrovasculaires et nerveuses, soulager les troubles hormonaux, immunologiques ou l'obésité, soutenir les traitements du diabète, du cancer et/ou des affections inflammatoires.

15. Composition selon les revendications 1 à 10, destinée à équilibrer le taux de lipides sanguin, prévenir ou réduire le risque de développement de changements, de troubles ou de maladies athérosclérotiques, développer et maintenir les fonctions cognitives, soulager et/ou prévenir les maladies des vaisseaux sanguins, les maladies cardiovasculaires, cérébrovasculaires et nerveuses, soulager les troubles hormonaux, immunologiques ou l'obésité, soutenir les traitements du diabète, du cancer et/ou des affections inflammatoires.
